(19) **Europäisches Patentamt**
**European Patent Office**
**Office européen des brevets**

(11) **EP 3 348 184 A1**

(12) **EUROPEAN PATENT APPLICATION**

(43) Date of publication:
**18.07.2018 Bulletin 2018/29**

(51) Int Cl.:
***A61B 5/00*** (2006.01)

(21) Application number: **18000013.5**

(22) Date of filing: **08.01.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **12.01.2017 JP 2017003393**

(71) Applicant: **CANON KABUSHIKI KAISHA Tokyo (JP)**

(72) Inventors:
• **BABA, Yoshitaka**
  **Tokyo (JP)**
• **ABE, Naoto**
  **Tokyo (JP)**
• **MIYASATO, Takuro**
  **Tokyo (JP)**

(74) Representative: **WESER & Kollegen**
  **Radeckestraße 43**
  **81245 München (DE)**

(54) **OBJECT INFORMATION ACQUIRING APPARATUS AND OBJECT INFORMATION ACQUIRING METHOD**

(57) An object information acquiring apparatus, comprises a signal acquiring unit that acquires a signal obtained by converting, with an acoustic element, an acoustic wave generated from an object to which light is irradiated; an information acquiring unit that performs, on the acquired signal, a signal process including at least any of an input impedance matching process, a filtering process, an amplification process, a subtraction process, and a signal extraction process, and acquire characteristic information inside the object using the processed signal; and a form acquiring unit that acquires form information of the object, wherein the information acquiring unit determines, based on the form information, contents of the signal process to be performed on a signal corresponding to an acoustic wave arriving from a target region.

FIG. 1

## Description

BACKGROUND OF THE INVENTION

Field of the Invention

The present invention relates to an apparatus for acquiring information in an object.

Description of the Related Art

Recently, in the field of medicine, research is underway on imaging of functional information of the inside of an object such as structural information and physiological information. Photoacoustic tomography (PAT) is recently being proposed as one of such imaging techniques.

When a living organism that is an object is irradiated with light such as laser light, an acoustic wave (typically, an ultrasonic wave) is generated as the light is absorbed by living tissue inside the object. This phenomenon is referred to as a photoacoustic effect and an acoustic wave generated by the photoacoustic effect is referred to as a photoacoustic wave. Since tissues constituting an object have respectively different absorption rates of optical energy, sound pressure of generated photoacoustic waves also differ. With PAT, by receiving a generated photoacoustic wave with a probe and mathematically analyzing a received signal, characteristic information inside the object can be acquired.

With an apparatus that acquires object information using an acoustic wave, an appropriate signal process is favorably performed in order to ensure accuracy of the information. For example, Japanese Patent Application Laid-open No. 2011-229815 discloses a technique for improving an S/N ratio by appropriately adjusting gain of an amplifier in accordance with a state of an object.

SUMMARY OF THE INVENTION

With an object information acquiring apparatus described in Japanese Patent Application Laid-open No. 2011-229815, maximum sound pressure of a photoacoustic wave generated from an object is measured and an amplification factor (gain) is set so that sound pressure does not become saturated.

An object information acquiring apparatus using a photoacoustic effect is capable of taking measurements of a blood vessel and, based on an obtained result, calculating oxygen saturation of blood and the like. However, in reality, there may be cases where signals are saturated due to an acoustic wave generated on the skin being stronger than an acoustic wave generated at a blood vessel inside a living organism and, consequently, a signal originating from a blood vessel cannot be accurately acquired. In order to solve this problem, a process may conceivably be performed which prevents a signal generated from a desired region (for example, a region in which a blood vessel is present) from becoming saturated. However, since the skin and a surface blood vessel exist in proximity of each other, it is difficult to determine a site from which a photoacoustic signal originates.

As described above, with a conventional object information acquiring apparatus, there is a problem in that, since an acoustic wave originating from the skin cannot be distinguished from an acoustic wave originating from a blood vessel, gain must be set low in order to prevent an acquired photoacoustic signal from becoming saturated. In addition, accordingly, there is a problem in that a signal originating from a blood vessel cannot be obtained with sufficient accuracy.

The present invention was devised in view of the foregoing problems.

The present invention in its first aspect provides an object information acquiring apparatus as specified in claims 1 to 15.

The present invention in its second aspect provides an object information acquiring method as specified in claims 16 to 25.

The present invention in its third aspect provides a non-transitory computer readable storing medium as specified in claim 26.

According to the present invention, an object information acquiring apparatus capable of acquiring highly accurate object information can be provided.

Further features of the present invention will become apparent from the following description of exemplary embodiments with reference to the attached drawings.

BRIEF DESCRIPTION OF THE DRAWINGS

FIG. 1 is a block diagram showing a configuration of an object information acquiring apparatus according to a first embodiment;

FIG. 2 is a diagram showing a configuration of a computer 700 in detail;

FIG. 3 is an operational flow chart of an object information acquiring apparatus according to the first embodiment;

FIGS. 4A to 4C are diagrams illustrating a positional relationship between acoustic wave receiving elements and an object;

FIG. 5 shows an example of a photoacoustic signal obtained by a simulation;

FIG. 6 is a diagram showing a configuration of an analog circuit section 735 in detail;

FIG. 7 is a diagram illustrating a write enable signal with respect to a FIFO 716;

FIGS. 8A and 8B are diagrams illustrating a check method of system noise;

FIG. 9 is a block diagram showing a configuration of an object information acquiring apparatus according to a third embodiment;

FIG. 10 is a diagram showing a configuration of a computer 1700 in detail;

FIG. 11 is an arrangement diagram of acoustic wave receiving elements according to the third embodiment;

FIG. 12 is an operational flow chart of an object information acquiring apparatus according to the third embodiment; and

FIG. 13 is a diagram illustrating timings at which ultrasonic measurement and photoacoustic measurement are performed.

## DESCRIPTION OF THE EMBODIMENTS

Hereinafter, preferred embodiments of the present invention will be described with reference to the drawings. However, it is to be understood that dimensions, materials, shapes, relative arrangements, and the like of components described below are intended to be changed as deemed appropriate in accordance with configurations and various conditions of apparatuses to which the present invention is to be applied. Therefore, the scope of the present invention is not intended to be limited to the embodiments described below.

The present invention relates to a technique for detecting an acoustic wave propagating from an object and generating and acquiring characteristic information of the inside of the object. Accordingly, the present invention can be considered an object information acquiring apparatus or a control method thereof, or an object information acquiring method and a signal processing method. The present invention can also be considered a program that causes an information processing apparatus including hardware resources such as a CPU and a memory to execute these methods, or a storage medium or an information processing apparatus that stores the program.

The object information acquiring apparatus according to the present invention includes an apparatus utilizing a photoacoustic effect in which an acoustic wave generated inside an object when the object is irradiated with light (an electromagnetic wave) is received, and characteristic information of the object is acquired as image data. In this case, characteristic information refers to information on a characteristic value corresponding to each of a plurality of positions inside the object which is generated using a received signal obtained by receiving a photoacoustic wave.

Characteristic information acquired by photoacoustic measurement is a value reflecting an absorption rate of optical energy. For example, characteristic information includes a generation source of an acoustic wave generated by light irradiation, initial sound pressure inside an object, an optical energy absorption density or an absorption coefficient derived from initial sound pressure, and a concentration of substances constituting tissue. In addition, a distribution of oxygen saturation can be calculated by obtaining a concentration of oxygenated hemoglobin and a concentration of reduced hemoglobin as concentrations of substances. Furthermore, a glucose concentration, a collagen concentration, a melanin concentration, a volume fraction of fat or water, and the like are also obtained.

The object information acquiring apparatus according to the present invention includes an apparatus utilizing ultrasonic echo technology which transmits an ultrasonic wave to an object, receives a reflected wave (an echo wave) that is reflected inside the object, and acquires object information as image data. In this case, the acquired object information is information reflecting a difference in acoustic impedances among tissues inside the object.

A two-dimensional or three-dimensional characteristic information distribution is obtained based on characteristic information at each position in the object. Distribution data may be generated as image data. Characteristic information may be obtained as distribution information of respective positions inside the object instead of as numerical data. In other words, distribution information such as a distribution of initial sound pressure, a distribution of energy absorption density, a distribution of absorption coefficients, and a distribution of oxygen saturation can be obtained.

An acoustic wave in the present specification is typically an ultrasonic wave and includes an elastic wave which is also referred to as a sonic wave or an acoustic wave. An electrical signal transformed from an acoustic wave by a probe or the like is also referred to as an acoustic signal. However, descriptions of an ultrasonic wave and an acoustic wave in the present specification are not intended to limit a wavelength of the elastic waves thereof. An acoustic wave generated by a photoacoustic effect is referred to as a photoacoustic wave or an optical ultrasonic wave. An electrical signal derived from a photoacoustic wave is also referred to as a photoacoustic signal. In addition, an electrical signal derived from an ultrasonic echo is also referred to as an ultrasonic signal.

(First Embodiment)

<System Configuration>

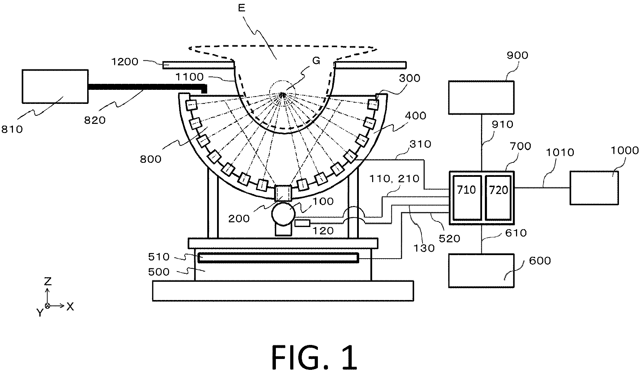

FIG. 1 is a functional block diagram showing a configuration of an object information acquiring apparatus according to a first embodiment.

The object information acquiring apparatus according to the first embodiment is a photoacoustic apparatus and includes a light source 100, a photodetector 120, an optical system 200, an acoustic wave receiving element 300, a supporter 400, a scanning section 500, and a scanning position sensor 510.

The object information acquiring apparatus according to the present embodiment further includes a form acquiring section 600, a computer 700, a liquid supplying/discharging unit 810, a display apparatus 900, an input section 1000, a form retaining section 1100, and a mounting section 1200.

<<Object E>>

Although an object E does not constitute a part of the object information acquiring apparatus according to the present invention, a description will be given below. The object information acquiring apparatus according to the present invention is an apparatus for diagnosing a malignant tumor, a vascular disease, a blood sugar level, and the like and performing a follow-up observation of chemotherapy of a human or an animal. Therefore, a living organism or, more specifically, a breast, a finger, a limb, and the like of a human or an animal are assumed as objects.

Since light absorbers with a large light absorption coefficient such as water, fat, protein, oxygenated hemoglobin, and reduced hemoglobin in the case of a living organism are present inside an object, a photoacoustic wave is generated due to light irradiation. When a phantom is used as an object, a photoacoustic wave can be generated and measured by sealing a substance simulating optical characteristics as a light absorber inside the phantom. Moreover, the object E is indicated by a dotted line in FIG. 1 for the sake of convenience.

<<Light Source 100>>

The light source 100 is an apparatus that generates pulse light for irradiating an object. While the light source is desirably a laser light source in order to obtain a large output, a light-emitting diode, a flash lamp, or the like may be used in place of a laser. When using a laser as the light source, various lasers such as a solid-state laser, a gas laser, a dye laser, and a semiconductor laser can be used.

In addition, desirably, a wavelength of the pulse light is a specific wavelength which is absorbed by a specific component among components constituting the object and which enables light to propagate to the inside of the object. Specifically, when the object is a living organism, light with a wavelength of at least 700 nm and not more than 1200 nm is desirably used. Since light in this range is capable of reaching relatively deep portions of the living organism, information of deep portions can be acquired. When limited to a measurement of surface portions of the living organism, a region from visible light to near infrared light with a wavelength of around 500 to 700 nm may be used.

In addition, in order to effectively generate a photoacoustic wave, light must be irradiated in a sufficiently short period of time in accordance with thermal characteristics of an object. When the object is a living organism, a pulse width of the pulse light generated by the light source is preferably equal to or less than several 10 nanoseconds.

Moreover, timings, waveforms, intensity, and the like of light irradiation are controlled by the computer 700 to be described later.

<<Optical System 200>>

The optical system 200 is a member that transmits pulsed light emitted from the light source 100. Light emitted from the light source is guided to and irradiates the object while being processed into a desired light distribution shape by an optical component such as a lens and a mirror. Moreover, light can also be propagated using an optical waveguide such as an optical fiber.

For example, the optical system 200 may include an optical device such as a lens, a mirror, a prism, an optical fiber, a diffuser plate, and a shutter. Any optical component may be used as long as light emitted by the light source can irradiate the object in a desired shape. Moreover, spreading light over a relatively wide area is more favorable than focusing light with a lens from the perspective of safety with respect to the object and the perspective of expanding a diagnostic area.

In the present embodiment, the optical system 200 is configured so as to irradiate a region of a center of curvature of an acoustic probe arranged in a hemispherical supporter 400 (to be described later).

Moreover, opening and closing of a shutter and the like in the optical system 200 is performed by the computer 700 (to be described later). In addition, the light source 100 and the optical system 200 are provided in a bottom part of the supporter 400 (to be described later) and move in accordance with the movement of the supporter 400.

<<Photodetector 120>>

The photodetector 120 is an apparatus for detecting that light has been irradiated from the light source 100. In the present embodiment, an optical fiber (not shown) included in the optical system 200 is partially branched and input to a photodiode, and a detected signal is transmitted to the computer 700. Moreover, any kind of apparatus may be used as the photodetector 120 as long as a timing of limit emission by the light source 100 can be detected. In addition, the detecting means is not limited to a photodiode.

<<Acoustic Wave Receiving Element 300>>

The acoustic wave receiving element 300 is means for receiving an acoustic wave arriving from inside the object E and converting the acoustic wave into an electrical signal. The acoustic wave receiving element is also referred to as a probe, an acoustic probe, an acoustic wave detector, an acoustic wave receiver, and a transducer.

Since acoustic waves generated by a living organism are ultrasonic waves ranging from 100 kHz to 100 MHz, an element capable of receiving this frequency band is used as the acoustic wave receiving element. Specifically, a transducer using a piezoelectric phenomenon, a transducer using optical resonance, a transducer using a variation in capacity, or the like can be used.

In addition, desirably, the acoustic element has high sensitivity and a wide frequency band. Specific examples of the acoustic element include a piezoelectric element using lead zirconate titanate (PZT) or the like, a polymer piezoelectric film material such as polyvinylidene fluoride (PVDF), a capacitive micromachined ultrasonic transducer (CMUT), and an acoustic element using a Fabry-Perot interferometer. However, the acoustic element is not limited to these examples and any acoustic element may be used as long as functions as a probe are fulfilled.

With the acoustic wave receiving element 300, receiving sensitivity is highest when a photoacoustic wave is incident from a normal direction of a receiving surface, and the larger the angle between the normal direction of the receiving surface and the angle of incidence of the photoacoustic wave, the lower the receiving sensitivity. Moreover, the acoustic wave receiving element 300 according to the present embodiment is assumed to have a receiving surface with a circular planar shape.

<<Supporter 400>>

The supporter 400 is an approximately hemispherical container that supports the optical system 200 and a plurality of the acoustic wave receiving elements 300. In the present embodiment, the plurality of acoustic wave receiving elements 300 are installed on an inside surface of the hemisphere and the optical system 200 is installed at a bottom part (a pole) of the hemisphere. In addition, the inside of the hemisphere is filled with an acoustic matching member 800 to be described later. The supporter 400 is favorably constructed using a metal material or the like with high mechanical strength in order to support these members.

The plurality of acoustic wave receiving elements 300 are arranged in an array on the hemispherical surface so that reception directions of the elements are oriented toward a center of curvature of the hemisphere. Moreover, FIG. 1 represents a section of the hemispherical supporter 400 cut along a central axis thereof. In addition, dashed-dotted lines that converge to a partial region inside the object E indicate reception directions of the acoustic wave receiving elements 300.

In this manner, each of the plurality of acoustic wave receiving elements 300 is arranged on the supporter 400 so as to be capable of receiving a photoacoustic wave generated in a specific region with high sensitivity. In the present embodiment, the specific region (a region G in FIG. 1) will be referred to as a "high sensitivity region".

When object information is acquired by arranging the plurality of acoustic wave receiving elements 300 in this manner, resolution is highest at the center of curvature of the hemisphere, and the further away from the center, the lower the resolution. In the present embodiment, a region from a point where highest resolution is obtained to a point where resolution that is half of the highest resolution is obtained is assumed to be the high sensitivity region.

Moreover, directions of highest sensitivity of the respective acoustic wave receiving elements need not necessarily intersect each other as long as a prescribed high sensitivity region can be formed. In other words, at least a part of the plurality of acoustic wave receiving elements 300 need only be arranged so as to be capable of receiving a photoacoustic wave generated in a prescribed region with high sensitivity.

<<Scanning Section 500>>

The scanning section 500 is an apparatus (a scanning mechanism) which changes a relative position of the supporter 400 with respect to the object E by moving the position of the supporter 400 in a three-dimensional direction. The scanning section 500 has a guiding mechanism, a driving mechanism, and the scanning position sensor 510 in each of the three directions of X, Y, and Z axes.

As shown in FIG. 1, since the supporter 400 is loaded on the scanning section 500, the guiding mechanism is favorably a linear guide or the like capable of supporting a large load. In addition, a lead screw mechanism, a linking mechanism, a gear mechanism, a hydraulic mechanism, or the like can be used as the driving mechanism. Furthermore, a driving force can be obtained with a motor or the like.

Moreover, the object E may be moved while fixing the supporter 400. When moving the object E, a configuration is conceivable in which the object E is moved by moving a supporting section (not shown) that supports the object E or by moving the mounting section 1200. Alternatively, both the object E and the supporter 400 may be made movable.

In addition, while movement is desirably performed continuously, the movement may be performed by repeating a certain step. Furthermore, while the scanning section 500 is desirably a motor-driven stage, a manually-moved stage may be used instead. However, a configuration is not limited to those described above and any configuration may be adopted as long as at least one of the object E and the supporter 400 can be moved.

<<Scanning Position Sensor 510>>

The scanning position sensor 510 is means for acquiring a position (coordinates) of the supporter 400. The scanning position sensor 510 acquires one-dimensional, two-dimensional, or three-dimensional position coordinate information in accordance with a configuration of the apparatus. The scanning position sensor 510 may be any kind of sensor as long as a position of the supporter 400 can be acquired. For example, a potentiometer using a linear scale, a magnetic sensor, an infrared sensor, an ultrasonic wave sensor, an encoder, a variable resistor, or the like can be used as the position sensor.

<<Form Acquiring Section 600>>

The form acquiring section 600 is means for acquiring form information of the object E. The form information of an object is information representing a position of a structure constituting the object. The form information of an object may be information representing a shape of a surface of the object (hereinafter, shape information) or information representing a structure inside the object (hereinafter, structural information).

The form information acquired by the form acquiring section 600 is transmitted to the computer 700 (a calculating section 710) to be described later.

As methods by which the form acquiring section 600 acquires form information, the following three methods will now be exemplified.

A first method involves storing shape information of the surface of the object in advance and reading and using the shape information. For example, information related to profiles of a plurality of objects may be stored in the form acquiring section 600 (or the computer 700) and appropriate information may be automatically or manually read. In addition, information representing a profile of the form retaining section 1100 (to be described later) may be handled as information representing a profile of a surface of an object.

A second method involves measuring a surface profile of the object E to acquire shape information.

For example, the form acquiring section 600 can acquire shape information of the object E by transmitting an ultrasonic wave to the object E and analyzing a reflected wave. In this case, the form acquiring section 600 may have a transducer that transmits and receives an ultrasonic wave to and from the object separately from the acoustic wave receiving elements 300. In addition, reception of a photoacoustic wave and transmission and reception of an ultrasonic wave for measuring a profile may be performed by configuring a part of the acoustic wave receiving elements 300 so as to be capable of transmitting an ultrasonic wave. Moreover, while the measurement of a profile of an object using a reflected wave may be performed by the form acquiring section 600, the computer 700 (the calculating section 710) may be configured to perform the measurement instead.

A third method involves acquiring a position of a blood vessel (a blood vessel profile) existing inside the object E by measurement.

For example, using the configuration described above, a portion where a blood flow exists inside an object can be estimated by ultrasonic measurement (Doppler echocardiography). Moreover, a portion where a blood flow exists may be acquired as two-dimensional information or as three-dimensional information. In addition, a state of change in a blood flow may be acquired in real time.

Moreover, when using Doppler echocardiography, a processing circuit of a separate system may be provided.

For example, a dedicated A/D converter with a wide dynamic range and high resolution may be provided.

Moreover, while examples of acquiring form information using an ultrasonic measurement have been described for the second and third methods, other means (such as an MRI) may be used instead. In addition, structural information inside an object may be acquired using, in combination, a contrast agent and an MRI, an X-ray CT, or other modalities capable of confirming a portion where a blood flow exists.

Furthermore, form information may be acquired using a camera or the like. For example, form information of an object can also be acquired using a three-dimensional measurement technique such as a stereoscopic method based on images captured from a plurality of directions.

In addition, the form acquiring section 600 may present a user of the apparatus with acquired form information. For example, acquired form information may be displayed (parallel display, superposition display, alternate display, or the like) together with an image taken by a camera, object information (functional information) generated by the apparatus, or the like.

Furthermore, the form acquiring section 600 may acquire a plurality of types of information. For example, shape information of the form retaining section 1100, shape information of a surface of an object, or structural information (information related to a blood vessel profile) inside the object may be acquired. In addition, information to be used may be selected from a plurality of pieces of acquired information.

<<Computer 700>>

The computer 700 is means which is responsible for controlling the object information acquiring apparatus according to the present embodiment and is also equipped with functions as signal acquiring unit.

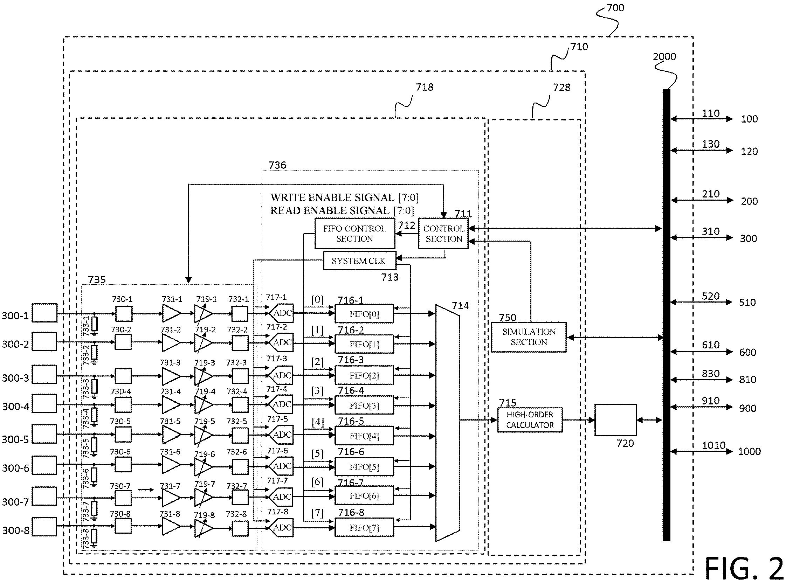

FIG. 2 is a diagram showing a configuration of the computer 700 in greater detail. As shown in FIG. 2, the computer 700 has the calculating section 710 and a storage section 720, and the calculating section 710 is constituted by a data acquiring section 718 and a calculation processing section 728. Furthermore, the data acquiring section 718 is constituted by an analog circuit section 735 and a digital circuit section 736.

The calculating section 710 is means for acquiring information inside an object by analyzing signals acquired from the plurality of acoustic wave receiving elements 300. In addition, the calculating section 710 has a function of simulating a waveform of a photoacoustic wave reaching the plurality of acoustic wave receiving elements 300 based on form information acquired by the form acquiring section 600 and determining contents of analog and digital signal processes using a result of the simulation. Accordingly, a photoacoustic wave arriving from a desired region inside the object can be processed by an optimal method. A specific method will be described later.

Hereinafter, the respective means constituting the calculating section 710 will be described.

The analog circuit section 735 is a circuit which subjects an electrical signal output from the plurality of acoustic wave receiving elements 300 to an analog signal process and which outputs a processed analog signal to the digital circuit section 736. Examples of a content of the signal process include a signal process using an analog circuit such as an input impedance matching process using an input impedance circuit, a filtering process using a filter, and an amplification process using an amplifier. However, the content of the analog signal process is not limited to these examples.

The digital circuit section 736 is a circuit which samples an analog signal input from the analog circuit section 735 and which generates a digital signal. In addition, the digital circuit section 736 is capable of performing a digital signal process on a digital signal such as applying a digital gain or applying a digital filter. However, the content of the digital signal process is not limited to these examples.

The calculation processing section 728 is constituted by a high-order calculator 715 and a simulation section 750.

The high-order calculator 715 is means for performing a process based on an image reconstruction algorithm on a digital signal generated by the data acquiring section 718 and acquiring object information. Due to the high-order calculator 715, information such as functional information of the surface or the interior of an object, oxygen saturation in an internal blood vessel of the object, and concentration of other blood components is acquired.

The high-order calculator 715 is typically constituted by a circuit such as a CPU, a GPU, an FPGA and an ASIC. Alternatively, the high-order calculator 715 may be constituted by a plurality of circuits. In addition, respective processes performed by the computer 700 may be executed by any of the circuits. Furthermore, the calculation processing section 728 may be configured so as to perform a part of all of the digital signal process to be performed by the digital circuit section 736.

The simulation section 750 is means for simulating, using form information of an object acquired by the form acquiring section 600, a waveform of a photoacoustic wave which is generated by the object having been irradiated with light and which reaches the plurality of acoustic wave receiving elements 300. The simulation section 750 also has a function of performing a Fourier analysis on the waveform of the acoustic wave and specifying a frequency component.

A simulation result generated by the simulation section 750 is fed back to the analog circuit section 735 and the digital circuit section 736 and used in a signal process performed by the respective circuits. Accordingly, the signal process of an appropriate content in accordance with the simulation result can be performed.

Moreover, while the simulation section 750 and the high-order calculator 715 are separated from each other in the present embodiment, the simulation section 750 and the high-order calculator 715 may be configured as a single module instead.

The storage section 720 is storage means typically constituted by a storage medium such as a ROM, a RAM, and a hard disk. Alternatively, the storage sections may be constituted by a plurality of storage media.

As described above, the calculating section 710 generates object information by subjecting an electrical signal output from the plurality of acoustic wave receiving elements 300 to both an analog signal process and a digital signal process.

In addition, the calculating section 710 is also responsible for controlling, via a bus 2000, operations of the respective elements constituting the object information acquiring apparatus. For example, the calculating section 710 controls a timing at which the light source 100 emits pulsed light.

Moreover, the computer 700 is favorably configured so as to be capable of simultaneously performing pipeline processes on a plurality of signals. Accordingly, a period of time required to acquire object information can be reduced. In addition, respective processes to be performed by the computer 700 can be stored in the storage section 720 as programs to be executed by the calculating section 710. In this case, the storage section 720 in which the programs are to be stored constitutes a non-transitory recording medium.

<<Acoustic Matching Member 800>>

The acoustic matching member 800 is a member for filling a space between the object E and the acoustic wave receiving elements 300 and acoustically coupling the object E and the acoustic wave receiving elements 300 with each other. The acoustic matching member 800 may be arranged between the acoustic wave receiving elements 300 and the form retaining section 1100 or between the form retaining section 1100 and an object. Moreover, when the acoustic matching member 800 is to be dispersed to a plurality of locations, a different member may be used at each location.

The acoustic matching member 800 is favorably a material with an acoustic impedance similar to those of the object and the acoustic wave receiving elements 300 (for example, an intermediate acoustic impedance of the object and the acoustic wave receiving elements 300). In addition, the acoustic matching member 800 is favorably a material which transmits pulse light generated by the light source 100. Furthermore, the acoustic matching member 800 is favorably a liquid. Typically, water, castor oil, a gel, and the like can be used.

<<Liquid Supplying/Discharging Unit 810>>

The liquid supplying/discharging unit 810 is a unit which supplies and discharges the acoustic matching member 800. The liquid supplying/discharging unit 810 supplies the acoustic matching member 800 into the supporter 400 at an appropriate timing prior to performing a measurement. In addition, once the acoustic matching member 800 is no longer required, the liquid supplying/discharging unit 810 collects the acoustic matching member 800 from the supporter 400. Moreover, since there is a possibility that internal humidity may rise and a defect may occur in the apparatus when the acoustic matching member 800 remains pooled in the supporter 400, the acoustic matching member 800 is desirably collected when the apparatus is not in use.

<<Display Apparatus 900>>

The display apparatus 900 is an apparatus which displays, using images, numerical values, and the like, object information output from the computer 700. While the display apparatus 900 is typically a liquid crystal display or the like, the display apparatus 900 may be a display adopting other systems such as a plasma display, an organic EL display, and an FED. In addition, .the display apparatus 900 may be provided separate from the object information acquiring apparatus according to the present embodiment.

<<Input Section 1000>>

The input section 1000 is means used by the user of the apparatus to input information to the computer 700. As the input section 1000, for example, a keyboard, a mouse, a touch panel, a dial, a button, and the like can be used. Alternatively, a touch panel can be adopted as the input section 1000, in which case the touch panel doubles as the display apparatus 900 and the input section 1000.

<<Form Retaining Section 1100>>

The form retaining section 1100 is a member which is abutted against an object in order to keeping a shape of the object constant.

The form retaining section 1100 is mounted to the mounting section 1200. Moreover, a plurality of form retaining sections may be made replaceable in accordance with a shape or a state of retention of the object. In this case, the mounting section 1200 is favorably configured so that a plurality of form retaining sections can be mounted to the mounting section 1200.

In the present embodiment, since the object E is irradiated with light via the form retaining section 1100, the form retaining section 1100 is favorably a member that transmits light. For example, polymethylpentene, polyethylene terephthalate, and the like can be preferably used.

Moreover, when the object is a breast, the breast is favorably retained in a state where deformation of the breast shape is reduced and a constant shape is attained. For this reason, the shape of the form retaining section 1100 is favorably a shape created by cutting a sphere at a given section. The shape of the form retaining section 1100 can be designed as appropriate in accordance with a volume of the object or a desired shape after retention of the object.

<Processing Flow Chart>

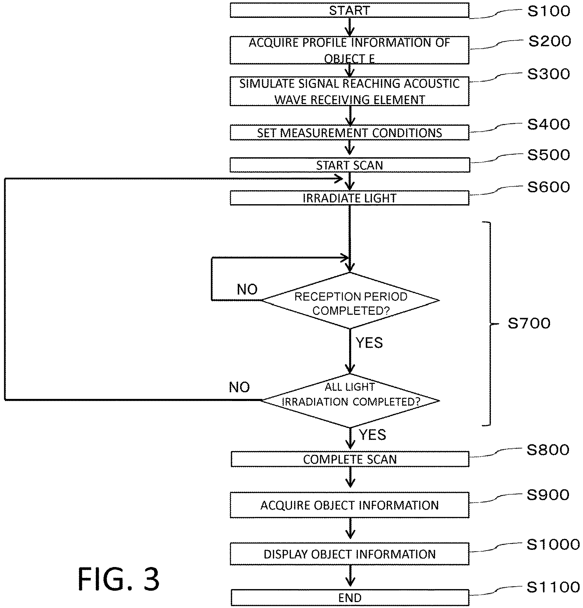

Next, operations of the object information acquiring apparatus according to the present embodiment will be described with reference to the flow chart shown in FIG. 3.

In step S100, a measurement is started. At this point, an object is inserted to the form retaining section 1100, and a space between the supporter 400 and the form retaining section 1100 and a space between the form retaining section 1100 and the object are filled with the acoustic matching member 800.

Next, in step S200, the form acquiring section 600 performs an ultrasonic measurement (an ultrasonic echo measurement) for acquiring form information of the object. In the present step, any of one-dimensional A-mode imaging, two-dimensional or three-dimensional B-mode imaging, and Doppler imaging is performed to obtain shape information regarding the object (for example, information regarding an external shape of the object) or structural information regarding the object (for example, information regarding a blood vessel profile inside the object). Performing the present step enables a positional relationship between skin and a surface blood vessel of the object to be comprehended.

Moreover, after acquiring shape information of the object, a process of estimating a position of a blood vessel by an arbitrary method may be performed.

Next, in step S300, based on the information acquired by the form acquiring section 600, the calculating section 710 (the simulation section 750) simulates a waveform of an acoustic wave which is generated by the object having been irradiated with light and which reaches the plurality of acoustic wave receiving elements 300.

A specific method of performing the simulation will now be described.

First, as a first step of the simulation, a distribution of a light amount reaching inside the object is calculated based on a distribution of light irradiated on the surface of the object (irradiation light distribution), an object shape, and an optical coefficient distribution of the object. Basically, the light amount distribution inside the object can be calculated using a diffusion equation such as Expression (1). Moreover, the diffusion equation to be used need not necessarily be the exemplified equation. For example, Expression (1) may be suitably deformed using an appropriate approximation or the like in order to obtain a three-dimensional distribution of light or to adjust an amount of calculations to a suitable amount.

[Math. 1]

$$\phi(r) = \frac{1}{4\pi D} \cdot \frac{\exp(-\mu_{\mathit{eff}} \cdot r)}{r} \quad \cdot \cdot \cdot \text{Expression (1)}$$

Next, the light amount distribution inside the object calculated in the previous step is multiplied by a distribution of absorption coefficients of the object and the Grüneisen parameter to calculate a distribution of initial sound pressure inside the object.

A relationship between the sound pressure of an acoustic wave created by a photoacoustic effect and light intensity inside the object will now be described. Sound pressure $p_0$ [Pa] of an acoustic wave created by a photoacoustic effect is represented by Expression (2).

[Math. 2]

$$p_0 = \mu_a \cdot \Gamma \cdot \Phi \quad \cdots \quad \text{Expression (2)}$$

In Expression (2), $\mu_a$ denotes an absorption coefficient [/mm] of a light absorber (a tumor or the like). $\Gamma$ denotes the Grüneisen parameter. $\Phi$ denotes light intensity [J/mm$^2$] at a position of the light absorber. The Grüneisen parameter $\Gamma$ is a value obtained by dividing, by specific heat capacity at constant pressure, a product of the volume thermal expansion coefficient and the square of the speed of sound and has an approximately constant value in the case of a living organism. As is apparent from Expression (2), the generated sound pressure is proportional to the intensity of light. In this case, a distribution of $p_0$ obtained by Expression (2) inside the object is the distribution of initial sound pressure $p_0(r)$ inside the object.

Next, the manner in which a photoacoustic wave generated inside the object propagates inside a living organism is calculated. Generally, the propagation of a photoacoustic wave that propagates through a non-viscous medium such as a living organism is represented by the following photoacoustic wave equation.

[Math. 3]

$$\frac{\partial^2 p(r,t)}{\partial t^2} - c(r)^2 \rho(r) \nabla \cdot \left( \frac{1}{\rho(r)} \nabla p(r,t) \right) = \Gamma \frac{\partial H(r,t)}{\partial t} \quad \cdots \quad \text{Expression (3)}$$

In Expression (3), $p(r, t)$ denotes sound pressure, $\rho(r)$ denotes density, $c(r)$ denotes the speed of sound, and $\Gamma$ denotes the Grüneisen parameter. In addition, $H(r, t)$ denotes a heat quantity per unit time and unit volume, $t$ denotes time, and $r$ denotes a position. The heat quantity $H(r, t)$ per unit time and unit volume can be separated into a heat quantity $H(r)$ per unit volume and a pulsed light function $I(t)$ under a thermal confinement condition.

[Math. 4]

$$H(r,t) = H(r)I(t) \quad \cdots \quad \text{Expression (4)}$$

In addition, the heat quantity $H(r)$ per unit volume and the distribution of generated initial sound pressure $p_0(r)$ are in a relationship represented below.

[Math. 5]

$$p_0(r) = \Gamma \mu_a(r)\Phi(r) = \Gamma H(r) \quad \cdots \quad \text{Expression (5)}$$

In this case, $\mu_a(r)$ denotes an absorption coefficient [/mm] of a light absorber (a tumor or the like) and $\Phi(r)$ denotes light intensity [J/mm$^2$] in the light absorber.

To summarize the result described above, a wave equation under conditions satisfying a thermal confinement condition is as follows.

[Math. 6]

$$\frac{\partial^2 p(r,t)}{\partial t^2} - c(r)^2 \rho(r) \nabla \cdot \left( \frac{1}{\rho(r)} \nabla p(r,t) \right) = p_0(r) \frac{\partial I(r,t)}{\partial t} \quad \cdots \quad \text{Expression (6)}$$

By solving this equation, a process of propagation of a photoacoustic wave can be reproduced. In other words, sound pressure $p(R, T)$ of a photoacoustic wave at time $T$ at a position $R$ where the acoustic wave receiving element 300 exists can be simulated.

Moreover, the wave equation used in the simulation is not necessarily limited to Expression (6). The propagation process of a photoacoustic wave may be calculated using Expression (3) suitably deformed or simplified by limiting conditions in order to adjust an amount of calculations to a suitable amount or by using an appropriate approximation or the like.

In addition, a target region on which a simulation is performed may be an entire measurement region of the object or may be narrowed down to a region in which a region of interest is present. Since the primary objective of the present invention is to distinguish between a photoacoustic signal originating from the skin and a photoacoustic signal originating from a region of interest such as a surface blood vessel, processing time can be reduced by performing a simulation only on a region of interest.

Moreover, not all processes required by a simulation need be performed by the simulation section 750. For example, (a part of or all of) a simulation may be performed by a separately provided external computer (not shown) and a result of the simulation may be retrieved therefrom. In this manner, by distributing the process, a calculation load on the simulation section 750 can be reduced.

In addition, while a simulation of an acoustic wave is performed by calculation in the present example, an approximation may be performed based on sample data. For example, a simulation result may be obtained using sample data of a signal waveform generated by a representative profile inside a living organism. According to this method, the amount of calculations can be reduced and the process can be accelerated. Moreover, the sample data may be stored inside the apparatus or may be acquired from outside of the apparatus.

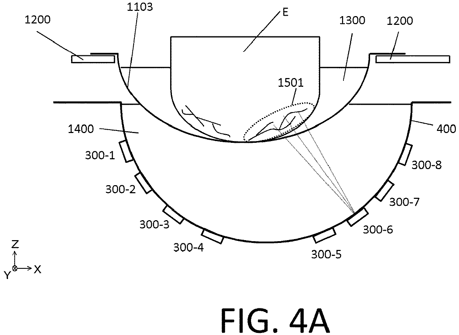

Next, the target region on which a simulation is performed will be described with reference to FIG. 4A.

As described above, a simulation may be performed on an entire measurement region or may only be performed on a region (for example, a region 1501) where a surface blood vessel is recognized to exist.

A simulation is performed in consideration of a band and a directionality of the plurality of acoustic wave receiving elements 300 by calculating a waveform of a photoacoustic wave which is generated from a surface blood vessel and which reaches the plurality of acoustic wave receiving elements 300. Moreover, as shown in FIG. 4A which represents a positional relationship between an acoustic wave receiving element 300-6, an object, and the region 1501 in which a surface blood vessel exists, a region on which a simulation is to be performed may be determined in consideration of a positional relationship between an element and a region of interest.

In addition, a simulation of a waveform of a photoacoustic signal which is generated on a surface (in other words, the skin) of the object and which reaches the plurality of acoustic wave receiving elements 300 may be performed at the same time. In doing so, color information of the skin of the object may be used as a parameter.

Accordingly, a waveform, intensity, and frequency characteristics of an acoustic wave which is generated from a surface blood vessel or skin and which reaches the plurality of acoustic wave receiving elements 300 can be predicted. In other words, since a timing at which an acoustic wave originating from a surface blood vessel reaches the plurality of acoustic wave receiving elements 300 and a waveform and intensity of the acoustic wave can be comprehended, conditions for extracting a photoacoustic signal originating from the surface blood vessel with high accuracy without saturating the photoacoustic signal can be accurately comprehended.

Methods of using a simulation result will be described with reference to step S700.

Next, in step S400, the user inputs measurement conditions. Specifically, for example, parameters are input such as a target region in which object information is to be acquired, an irradiation interval of light, a wavelength of light, the number of times light is to be irradiated, a speed of movement of the supporter 400, an absorption coefficient, a scattering coefficient, and a period during which photoacoustic waves generated inside the object is to be acquired (hereinafter, a reception period).

Moreover, in the present step, band characteristics of a filter, an input impedance value, a gain value, a sampling clock frequency, and the like may be input while referring to a result of a simulation. In addition, parameters representing the measurement conditions may be input manually or may be configured to be automatically input based on a result of a simulation.

Next, in step S500, a scan is started.

When the scan is started, the computer 700 outputs a control signal so that light is emitted at a prescribed timing from the light source 100. The emitted light is guided by the optical system 200 and irradiated on the object E via the acoustic matching member 800 (step S600). In addition, light irradiated on the object E is absorbed inside the object E and a photoacoustic wave is generated.

In step S700, the data acquiring section 718 existing inside the computer 700 acquires a photoacoustic signal during a reception period specified by the user. In addition, when a plurality of times are specified as the number of times light is to be irradiated, photoacoustic signals corresponding to the number of times are acquired.

<<Signal Process Based on Simulation Result>>

Contents of the signal process based on a simulation result will now be described.

An analog signal obtained by converting an acoustic wave received by the acoustic wave receiving element 300 is processed by the data acquiring section 718 and converted into a digital signal.

For example, the analog circuit section 735 which processes analog signals has a circuit such as those described below.

- An input impedance circuit which matches an impedance with the acoustic wave receiving element 300 and which prevents waveform deterioration attributable to reflection of the analog signal
- A filter circuit which removes a high-frequency component or a DC component of the analog signal
- An amplifier circuit which amplifies the analog signal

- An A/D converter circuit which converts the analog signal into a digital signal

A voltage value of an analog signal output from the acoustic wave receiving element 300 varies time-sequentially in accordance with a time-sequential variation of sound pressure of the received photoacoustic signal. In addition, voltage of the analog signal is divided by the input impedance circuit section, an unwanted component of the analog signal is removed by the filter circuit, the analog signal is amplified by the amplifier circuit, and the analog signal is sampled at a prescribed clock frequency and converted into a digital signal by the A/D converter circuit.

Conditions for accurately acquiring a signal originating from a surface blood vessel without saturating a photoacoustic signal will now be described.

In the present example, a case where an allowable input voltage range of an A/D converter is 2Vp - p in a receiving circuit that receives photoacoustic signals is assumed. In order to prevent saturation of a signal, an input impedance value (change an amplitude of analog signal by dividing voltage) or an amplification factor of a preamplifier must be determined such that an amplitude (a peak-to-peak value) of a photoacoustic signal does not exceed 2 V (does not saturate the analog signal). In addition, when a filtering process is to be performed in the receiving circuit, passband characteristics of a filter must be determined so that important characteristics among frequency characteristics of the photoacoustic signal do not deteriorate.

Due to the simulation, a timing at which an acoustic wave originating from a surface blood vessel reaches the plurality of acoustic wave receiving elements 300 and a waveform and intensity of the photoacoustic signal can be predicted. In addition, frequency band characteristics of a photoacoustic wave which originates from a surface blood vessel and which reaches the plurality of acoustic wave receiving elements 300 can also be predicted. Therefore, contents (optimal setting values such as an input impedance, a filter band, and a gain) of the process to be performed in the receiving circuit can be determined in order to acquire a desired photoacoustic wave (a photoacoustic wave originating from a surface blood vessel) without saturating the photoacoustic wave.

Information to be referred to in order to determine these conditions may be waveform information (a peak value and frequency characteristics of a waveform) of a photoacoustic signal or other information obtained as a result of a simulation. As a result, a photoacoustic signal corresponding to a received acoustic wave can be sampled without saturating the photoacoustic signal (in other words, without any deterioration or loss of information of the photoacoustic signal).

Moreover, information obtained from a simulation can also be utilized during image reconstruction. This will be described with reference to step S900.

Next, a process of extracting a signal originating from a region of interest from an acquired photoacoustic signal based on a simulation result will be described.

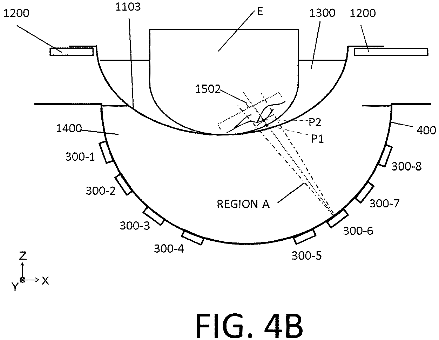

FIG. 4B shows an example of a reception status of a photoacoustic signal. A region A indicated by a dashed-dotted line schematically represents a range of directionality of the acoustic wave receiving element 300-6. In the case of the example shown in FIG. 4B, with respect to the acoustic wave receiving element 300-6, an acoustic wave originating from a point P1 on an object surface (in other words, the skin) reaches the acoustic wave receiving element 300-6 before an acoustic wave originating from a point P2 on a surface blood vessel. In other words, an acoustic wave originating from the point P1 and an acoustic wave originating from the point P2 reach the acoustic wave receiving element 300-6 at different timings.

Therefore, in consideration of characteristics of an acoustic wave originating from a surface blood vessel obtained from a simulation, a photoacoustic wave need only be received and processed after setting appropriate conditions which prevent saturation of a photoacoustic signal originating from a surface blood vessel and which enable the photoacoustic signal to be accurately acquired.

However, the situation changes when the range of directionality of the acoustic wave receiving element 300-6 changes.

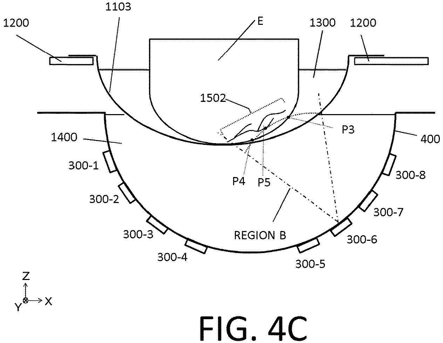

FIG. 4C is a diagram showing a case where the range of directionality of the acoustic wave receiving element 300-6 is wider than in the example shown in FIG. 4B. A region B indicated by a dashed-dotted line schematically represents a range of directionality of the acoustic wave receiving element 300-6. Compared to FIG. 4B, the range of directionality of the acoustic wave receiving element 300-6 has expanded. In this example, acoustic waves originating from points P3 and P4 on an object surface (in other words, the skin) reaches the acoustic wave receiving element 300-6 at the same time as an acoustic wave originating from a point P5 on a surface blood vessel. In other words, acoustic waves originating from the respective points reach the acoustic wave receiving element 300-6 in an added state.

In such a case, a process of extracting a photoacoustic wave signal originating from the point P5 need only be performed after temporarily acquiring a photoacoustic signal under conditions that do not impair characteristics of acoustic waves (added acoustic waves) originating from the points P3, P4, and P5.

The signal extraction process may be performed by the data acquiring section 718 or may be performed in a later stage (step S900) by the calculation processing section 728, which will be described now.

<<Example of Extracting Photoacoustic Signal from Digital Signal>>

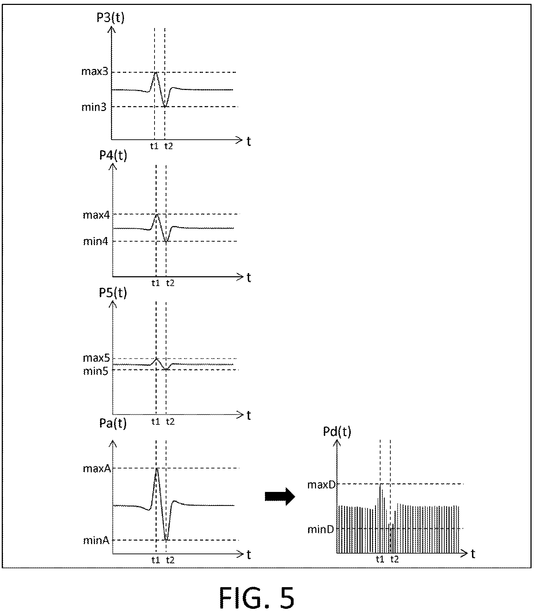

FIG. 5 shows an example of a photoacoustic signal obtained by a simulation in an example shown in FIG. 4C. In this case, it is assumed that waveform information P3(t), P4(t), and P5(t) of photoacoustic signals (analog signal waveforms output from the acoustic wave receiving elements 300) respectively originating from points P3, P4, and P5 have been obtained. It is also assumed that waveform information Pa(t) of a photoacoustic wave obtained by adding the photoacoustic waves originating from the points P3, P4, and P5 has been obtained (t denotes time).

In this case, an input impedance value and a gain value which prevent saturation of the waveform Pa(t) and a filter band which does not impair a frequency band of Pa(t) are set from a maximum value maxA of Pa(t), and a waveform of Pa(t) is sampled to obtain a digital signal Pd(t).

Subsequently, based on the waveform information P3(t), P4(t), and P5(t) of photoacoustic signals originating from the points P3, P4, and P5 and on the information of Pa(t), a waveform originating from the region of interest (in the present example, the point P5) is extracted from the digital signal Pd(t).

Moreover, digital signal components P3d(t), P4d(t), and P5d(t) of P3(t), P4(t), and P5(t) included in the digital signal Pd(t) can be calculated in accordance with proportions of signal amplitudes of P3(t), P4(t), and P5(t) in a signal amplitude of Pa (t) .

For example, a digital signal component P5d(t1) originating from P5 included in a digital signal Pd(t1) at time t1 can be calculated as follows.

$$P5d(t1) = Pd(t1) \times \{max5/(max3 + max4 + max5)\} =$$
$$Pd(t1) \times \{max5/(maxA)\}$$

This is because an amplitude value Pa(t1) = maxA of Pa(t) at the time t1 is represented as a sum of amplitude values max3, max4, and max5 of P3(t), P4(t), and P5(t) at the time t1.

In addition, a digital signal component P5d(t2) originating from P5 included in a digital signal Pd(t2) at time t2 can be calculated as follows.

$$P5d(t2) = Pd(t2) \times \{min5/(min3 + min4 + min5)\} =$$
$$Pd(t2) \times \{min5/(minA)\}$$

This is because an amplitude value Pa(t2) = minA of Pa(t) at the time t2 is represented as a sum of amplitude values min3, min4, and min5 of P3(t), P4(t), and P5(t) at the time t2.

In this manner, even when signals originating from a plurality of points reach an acoustic wave receiving element at the same time, a signal waveform originating from a region of interest can be extracted with good quantitative reliability from a digital signal based on prior information obtained from a simulation.

<<Example of Extracting Photoacoustic Signal from Analog Signal>>

A signal waveform originating from a region of interest can also be extracted from an analog signal based on prior information obtained from a simulation.

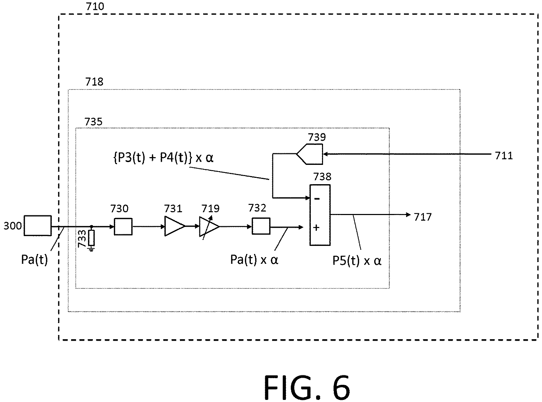

FIG. 6 shows the analog circuit section 735 which extracts a signal waveform originating from a region of interest from an analog signal. The analog circuit section 735 shown in FIG. 6 is constituted by an input impedance circuit 733, filters 730 and 732, a low noise amplifier (hereinafter, an LNA) 731, and a variable gain amplifier (hereinafter, a VGA) 719. The analog circuit section 735 is additionally constituted by a D/A converter 739 and a subtractor 738.

For example, when an analog electrical signal waveform Pa(t) output by the acoustic wave receiving element 300 is processed by a filter and amplified $\alpha$-number of times, a value $\{P3(t) + P4(t)\} \times \alpha$ obtained by similarly amplifying a sum of P3(t) and P4(t) $\alpha$-number of times is subtracted from Pa(t).

Accordingly, P5(t) can be extracted by a subtraction process of the analog signal as expressed below.

$$Pa(t) \times \alpha - \{P3(t) + P4(t)\} \times \alpha$$

$$= \{P3(t) + P4(t) + P5(t)\} \times \alpha - \{P3(t) + P4(t)\} \times \alpha$$

$$= P5(t) \times \alpha$$

By adding the subtractor 738 and the D/A converter 739 as shown in FIG. 6 to the exemplified configuration of the computer 700, a signal waveform originating from a region of interest can be extracted from an analog signal.

For example, digital signal data P3_4dg(t) output by a control section 711 which corresponds to an analog signal P3_4ag(t) (= {P3(t) + P4(t)} $\times \alpha$) is converted into an analog signal by the D/A converter 739 to reproduce P3_4ag(t). In addition, using the subtractor 738, P3_4ag(t) is subtracted from Pa(t) $\times \alpha$ to obtain P5(t) $\times \alpha$.

Performing such a process also enables a signal waveform originating from a region of interest to be extracted from an analog signal based on prior information obtained from a simulation. Moreover, an analog signal subtraction process need not necessarily be performed on an amplified analog signal. A signal subtraction process may be performed on a signal prior to being amplified and, when an analog signal is to be amplified a plurality of times, a signal subtraction process may be performed in an arbitrary stage among a plurality of amplification stages. A subtraction process of an analog signal may be performed in any way as long as an object signal can be appropriately extracted.

Let us now return to the description of step S700.

<<Details of Acquisition Process of Photoacoustic Signal>>

Details of a process of acquiring a photoacoustic signal in step S700 will now be described with reference to FIG. 2. Moreover, while a case where eight acoustic wave receiving elements are provided will be described in the present example, the number of acoustic wave receiving elements is not limited thereto. As described earlier, the data acquiring section 718 is constituted by the analog circuit section 735 and the digital circuit section 736.

First, a process performed by the analog circuit section 735 will be described.

The analog circuit section 735 is constituted by input impedance circuits 733-1 to 733-8, filters 730-1 to 730-8, low noise amplifiers 731-1 to 731-8, gain amplifiers 719-1 to 719-8, and filters 732-1 to 732-8.

In addition, the digital circuit section 736 is constituted by A/D converters (hereinafter, ADCs) 717-1 to 717-8, FIFOs 716-1 to 716-8, a clock supplying section 713 (depicted as SYSTEM CLK in the drawings), a FIFO control section 712, the control section 711, and a select switch 714.

Moreover, in the following description, the hyphen and thereafter in a reference numeral will be omitted when components need not particularly be distinguished from one another.

Next, an operation by which the data acquiring section 718 acquires a photoacoustic signal will be described.

First, the plurality of acoustic wave receiving elements 300 receive a photoacoustic wave, convert the photoacoustic wave into an electrical signal, and input the electrical signal to the filter 730.

The input impedance circuit 733 divides voltage of each output signal in accordance with a relationship of the plurality of acoustic wave receiving elements 300 with an output impedance value, and inputs the voltage-divided output signal to the filter 730.

In addition, the filter 730 performs a filtering process on the electrical signal and outputs the processed electrical signal to the LNA 731. The electrical signal amplified by the LNA 731 is output to the VGA 719. In addition, the electrical signal amplified by the VGA 719 is output to the filter 732, and the electrical signal subjected to a filtering process is output to the ADC 717 to be converted into a digital signal.

Moreover, while the input impedance circuit 733 may have a certain fixed impedance value, the input impedance circuit 733 may be configured such that an arbitrary impedance can be set by the control section 711. Alternatively, a plurality of input impedance values may be prepared so as to be selectable, and a specific input impedance value may be selected and set according to an instruction from the control section 711.

The filters 730 and 732 are high-pass filters for removing a DC component of a signal input from the plurality of acoustic wave receiving elements 300 and removing a frequency component that is lower than a band of each acoustic wave receiving element. In addition, the filters 730 and 732 are low-pass filters for removing loopback noise (aliasing) of sampling and removing a frequency component that is higher than a band of each acoustic wave receiving element.

For example, while the filter 730 may be a low-pass filter and the filter 732 may be a high-pass filter, the configuration of the filters need not necessarily be limited to the exemplified configuration. For example, instead of separately providing a high-pass filter and a low-pass filter, both a low frequency component and a high frequency component may be removed using a bandpass filter.

For example, when a sampling frequency is 20 MHz, the sampling theorem requires that a cutoff frequency of the low-pass filter be equal to or lower than 10 MHz. A cutoff frequency of the high-pass filter must be set while being careful not to remove signals within a band of the plurality of acoustic wave receiving elements 300.

Moreover, while cutoff frequencies of the filters 730 and 732 may be fixed, the filters 730 and 732 may be configured such that arbitrary cutoff frequencies can be set by the control section 711. Alternatively, a plurality of cutoff frequencies may be prepared so as to be selectable, and a cutoff frequency may be selected and set according to an instruction from the control section 711.

The LNA 731 and the VGA 719 amplify an electrical signal output by the plurality of acoustic wave receiving elements 300. Amplification factors of the LNA 731 and the VGA 719 are set by the control section 711.

This concludes the description of the signal process in the analog circuit section 735.

Next, a process performed by the digital circuit section 736 will be described.

The ADC 717 samples an electrical signal at a certain frequency in accordance with a clock output by the clock supplying section 713, converts the electrical signal into a digital signal, and outputs the converted digital signal to the FIFO memory (a first-in first-out memory: hereinafter, a FIFO) 716. In accordance with the clock output by the clock supplying section 713 and a write enable signal output by the FIFO control section 712, the FIFO 716 stores the digital signal output by the ADC 717.

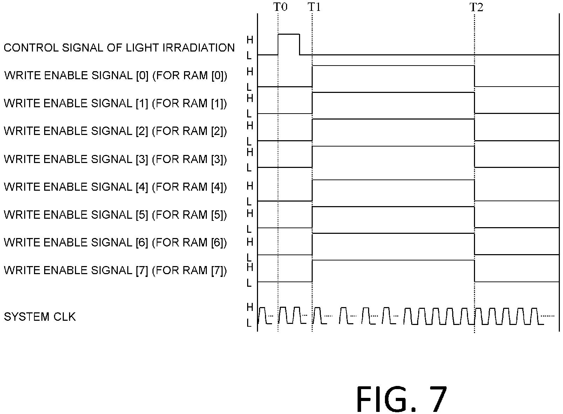

Based on a reception period parameter acquired in step S200, for example, the FIFO control section 712 adjusts an output timing of a write enable signal [7:0] with respect to the FIFO 716 as shown in FIG. 7. In FIG. 7, when a level of the write enable signal is low, write to the FIFO 716 is not performed, but when the level of the write enable signal is high, write to the FIFO 716 is performed. In the example shown in FIG. 7, a period from T1 to T2 which exists after a timing T0 where light irradiation is performed is a period in which a photoacoustic signal is being written into the FIFO 716.

Moreover, a frequency of a clock (a sampling clock) output by the clock supplying section 713 can also be set in advance by the user.

In accordance with a clock output by the clock supplying section 713 and a read enable signal output by the FIFO control section 712, the FIFO 716 transfers a stored digital signal to the high-order calculator 715.

The select switch 714 selects one of the FIFOs 716-1 to 716-8 and connects the selected FIFO to the high-order calculator 715, and transfers the digital signal into the high-order calculator 715. In addition, by repeating the selection of a FIFO to be connected to the high-order calculator 715 and the transfer of the digital signal to the high-order calculator 715, all digital signals stored in the FIFOs 716-1 to 716-8 are transferred. Moreover, a transfer destination of the digital signals need not necessarily be the high-order calculator 715 and may instead be the storage section 720. The digital signals may be transferred to any destination as long as the high-order calculator 715 can access the digital signals at appropriate timings.

In addition, while a configuration in which the select switch 714 selects one of the FIFOs 716-1 to 716-8 and transfers a digital signal to the selected FIFO is shown in FIG. 2, this configuration is not necessarily restrictive. For example, digital signals stored in a plurality of or all of the FIFOs 716-1 to 716-8 may be simultaneously transferred to the high-order calculator 715. Configurations are not limited to a specific configuration as long as the digital signals stored in the FIFOs 716-1 to 716-8 are appropriately transferred to the high-order calculator 715.

As described above, in step S700, electrical signals output from the plurality of acoustic wave receiving elements 300 are processed by the analog circuit section 735 and the digital circuit section 736 and stored as digital signals.

Moreover, while a photoacoustic signal is written to the FIFO 716 by a write enable signal in the present embodiment, this method is not necessarily restrictive. For example, after adopting a configuration in which a clock is constantly supplied to the FIFO 716, a clock may be supplied to the ADC 717 only when acquiring a photoacoustic signal. Alternatively, after adopting a configuration in which a clock is constantly supplied to the ADC 717, a clock may be supplied to the FIFO 716 only when acquiring a photoacoustic signal. Alternatively, a clock may be supplied to both the FIFO 716 and the ADC 717 only when acquiring a photoacoustic signal.

In addition, while a FIFO is exemplified as means for storing a digital signal output by the ADC 717 in the present embodiment, this is not necessarily restrictive. For example, a random access memory or the like may be used.

Moreover, a connection switch (not shown) may be provided between the plurality of acoustic wave receiving elements 300 and the analog circuit section 735 and signals may be acquired while switching connections between the plurality of acoustic wave receiving elements 300 and the analog circuit section 735. In this case, the number of channels of the analog circuit section 735 can be made smaller than the number of the plurality of acoustic wave receiving elements 300 and downsizing of the apparatus can be realized.

In addition, a connection switch (not shown) may be provided between the analog circuit section 735 and the digital circuit section 736, and a connection with an output of the analog circuit section 735 and a connection with the ADC 717 of the digital circuit section 736 may be switched. For example, a plurality of outputs of the analog circuit section 735 are connected to one of the ADCs 717 of the digital circuit section 736 via a connection switch (not shown).

In addition, connection destinations of the output of the analog circuit section 735 and the ADC 717 are switched at a frequency equal to or more than several times a sampling wave number so that sampling is performed at staggered timings in one sampling period. Accordingly, reception can be performed with a smaller number of ADCs 717 than the number of channels prepared for the analog circuit section 735.

This concludes the description of the process in step S700.

Once data acquisition under measurement conditions set in advance is completed, the scan is completed (step S800).

Next, in step S900, the calculation processing section 728 applies an image reconstruction algorithm to the digital signal acquired in step S700 to acquire information related to optical characteristics (for example, a distribution of absorption coefficients and oxygen saturation: referred to as object information) of the object.

As the image reconstruction algorithm for acquiring object information, a back projection method in a time domain or a Fourier domain which is commonly used in tomographic technology is known. Moreover, when more time can be allotted to image reconstruction, an image reconstruction method such as an inverse problem analysis method based on repetitive processes can also be used. The image reconstruction algorithm is not limited to a specific range as long as desired image reconstruction can be performed.

With the object information acquiring apparatus according to the present embodiment, a concentration ratio of Hb to $HbO_2$ contained in blood in body tissue is measured using a plurality of wavelengths to calculate oxygen saturation.

When acquiring oxygen saturation, a signal corresponding to a portion pertinent to a surface blood vessel of the object is desirably extracted with accuracy. As described earlier, calculating a frequency band of a signal originating from a surface blood vessel by simulation in advance enables a filter having passband characteristics matching the frequency band to be applied to a signal corresponding to a portion in which a surface blood vessel exists. Alternatively, an image processing filter may be applied to the portion in which a surface blood vessel exists. Consequently, a signal originating from a surface blood vessel can be accurately extracted and an image with superior visibility can be generated.

Moreover, when acquiring object information, a received photoacoustic signal is favorably corrected based on a result of the simulation performed in step S300.

For example, when a different sampling frequency is set for each of the plurality of acoustic wave receiving elements 300, digital signals to be used in image reconstruction must be appropriately selected in consideration of the difference in sampling frequencies.

In addition, when a different input impedance is applied to each of the plurality of acoustic wave receiving elements 300, signal levels at the ADC 717 respectively change due to the difference in input impedances. In consideration thereof, a digital signal corresponding to each of the plurality of acoustic wave receiving elements 300 may be amplified as appropriate after A/D conversion by the ADC 717.

In addition, when different gains are applied at the LNA 731 and the VGA 719 to each of the plurality of acoustic wave receiving elements 300, a process of correcting the difference in gains may be added after A/D conversion by the ADC 717. Accordingly, gains can be made substantially the same for all of the plurality of acoustic wave receiving elements 300. When a gain value remains varied for each of the plurality of acoustic wave receiving elements 300, quantitative reliability of the acquired form information and functional information deteriorates. By correctly comprehending the gain value corresponding to each of the plurality of acoustic wave receiving elements 300 and performing a process of correcting a variation among the gain values, a more accurate measurement of a concentration ratio can be performed.

Furthermore, a process using an inverse function of a transfer function of a "system" which couples the plurality of acoustic wave receiving elements 300 and the analog circuit section 735 (the input impedance circuit 733, the filter 730, the filter 732, the LNA 731, the VGA 719, and the ADC 717) can also be performed. For example, there is a process known as deconvolution which, using the inverse function of the transfer function, restores a waveform of an analog photoacoustic signal received by the plurality of acoustic wave receiving elements 300 from an acquired digital signal.

In such a case, when the transfer function changes in accordance with a reception condition setting of the analog circuit section 735, since the corresponding inverse function also changes, a result of a restoration process of a signal waveform also changes. When the transfer function changes, performing a restoration process of a signal waveform while fixing the corresponding inverse function without any consideration for the change in the inverse function prevents a correct restoration process from being performed. In consideration thereof, favorably, the transfer function and the inverse function of the analog circuit section 735 are derived and a restoration process of a signal waveform is accurately performed every time set conditions of the analog circuit section 735 are updated.

Next, in step S900, the display apparatus 900 displays the acquired object information and the process is finished (step S1100).

As described above, with the object information acquiring apparatus according to the first embodiment, firstly, an appropriate process can be performed on a photoacoustic signal prior to being subjected to reconstruction by distinguishing between a photoacoustic wave originating from skin and a photoacoustic wave originating from a surface blood vessel. Secondly, a signal originating from a surface blood vessel can be extracted. Accordingly, a calculation of object information can be accurately performed.

Moreover, while only a photoacoustic image is acquired in the present embodiment, acquisition of a photoacoustic image and acquisition of an ultrasonic image may be performed simultaneously and, when displaying object information, the images may be displayed superimposed or displayed side by side. Alternatively, the photoacoustic image and the ultrasonic image may be individually displayed.

In addition, conditions for processing a photoacoustic signal need not be the same with respect to all of the plurality of acoustic wave receiving elements 300 and may be individually set for each one of the plurality of acoustic wave receiving elements. Alternatively, same conditions may be collectively assigned to a certain number of elements. In other words, the plurality of acoustic wave receiving elements 300 may be divided into a plurality of groups and conditions may be individually assigned to each group.

Furthermore, when filters (the filters 730 and 732) with different band characteristics are applied to each of the plurality of acoustic wave receiving elements 300, acoustic wave receiving elements on which filters with same band characteristics are used may be grouped to be individually subjected to image reconstruction.

In addition, while a signal waveform originating from a region of interest is extracted based on prior information obtained by a simulation in the present embodiment, a method of separating and extracting a signal waveform originating from a region of interest from a digital signal waveform itself may be adopted instead. For example, a method of separating a superimposed signal such as singular spectrum analysis, independent component analysis, and principal component analysis, a method of estimating a signal waveform originating from a region of interest based on a statistical signal processing method, a signal extraction method based on a multivariate analysis technique, or wavelet transform may be applied. Furthermore, these methods may be applied in combination.

In addition, a method of separating and extracting photoacoustic waves respectively originating from inside and outside of a region of interest need only be capable of appropriately separating and extracting a photoacoustic wave originating from a point of a surface blood vessel inside an object and is not limited to a specific method.

Furthermore, while an example of distinguishing a photoacoustic wave originating from the surface (in other words, the skin) from a photoacoustic wave originating from a point of a surface blood vessel inside an object has been described in the present embodiment, this example is not restrictive. For example, a photoacoustic wave can be separated and extracted by a similar method even when photoacoustic waves respectively originating from different points on a surface blood vessel inside the object reach the acoustic wave receiving elements 300 at the same time. Accordingly, quantitative reliability of a measurement of blood concentration components such as localized oxygen saturation in a surface blood vessel inside the object may be improved.

At any rate, in a case where directionality is abnormally wide, a case where a side lobe occurs in the directionality, and the like, quantitative reliability of a measurement of blood concentration components may be improved by considering setting conditions for receiving photoacoustic waves and a method of processing after reception.

(Second Embodiment)

In a photoacoustic apparatus, favorably, whether or not system noise of the apparatus is appropriately maintained is monitored. This is because, when system noise increases due to some kind of failure, an S/N ratio of an acquired photoacoustic signal becomes degraded and a defect such as deterioration of quantitative reliability of functional information occurs. A second embodiment is an embodiment for measuring system noise of an apparatus in order to respond to such situations.

Moreover, system noise refers to data acquired by the data acquiring section 718 at timings where a measurement of an object is not performed. In an object information acquiring apparatus according to the second embodiment, the computer 700 is further equipped with a function of measuring system noise.

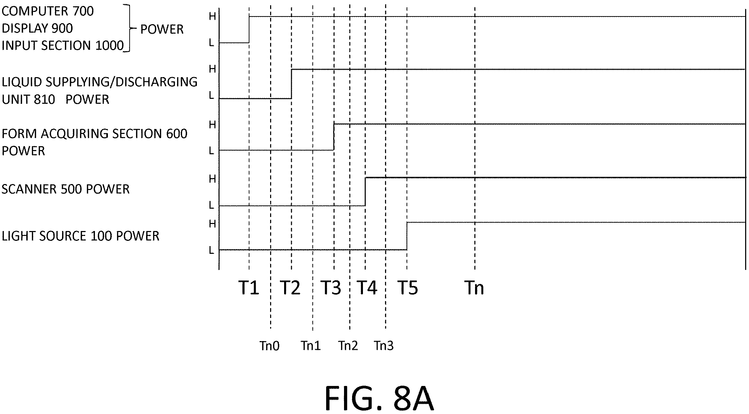

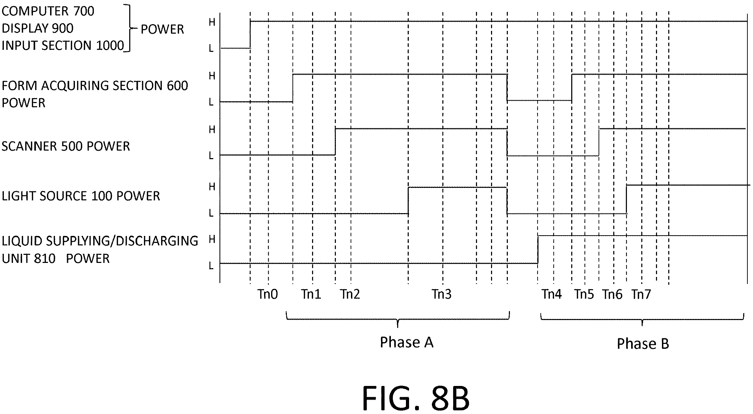

A method of checking system noise with the object information acquiring apparatus according to the present embodiment will be described with reference to the timing charts shown in FIGS. 8A and 8B. Moreover, in FIGS. 8A and 8B, H denotes a state where power of each module constituting the object information acquiring apparatus is turned on and L denotes a state where power is turned off.

As shown in FIG. 8A, a measurement of system noise can be performed after power to all of the constituent modules of the apparatus has been turned on and at a timing Tn where a photoacoustic wave is not being received.

Alternatively, a measurement may be performed as needed while sequentially turning on power of the respective constituent modules. For example, measurements may be performed at timings such as Tn0, Tn1, Tn2, and Tn3. When a large noise occurs due to a defect in any of the constituent modules and causes system noise to deteriorate, performing measurements in this manner makes it easier to specify in which constituent module the defect had occurred.

Alternatively, as shown in FIG. 8B, in a state where power to a specific constituent module is turned off, a measurement may be performed by sequentially turning on power to the other constituent modules. FIG. 8B represents an example of a case where measurements are separately performed in a state where power of a liquid supplying/discharging unit is turned off (Phase A) and in a state where power of the liquid supplying/discharging unit is turned on (Phase B). Accordingly, a change in system noise due to injection of the acoustic matching member 800 into the supporter 400 can be confirmed.

Moreover, while power of the constituent modules are sequentially turned on in the examples shown in FIGS. 8A and 8B, measurements may be respectively performed in a state where power of only one of the constituent modules is turned on. Accordingly, an effect of each constituent module on system noise can be confirmed.

In addition, when a capacitance type element such as a CMUT is used as the plurality of acoustic wave receiving elements 300, a plurality of voltages are applied to the element. In this manner, in a case of a system in which power needs to be supplied to each acoustic wave receiving element, a system noise measurement may be performed while controlling power supply for each element. Accordingly, an effect of each of the plurality of acoustic wave receiving elements on system noise can be confirmed.

Moreover, a method of measuring system noise is not limited to those described above. In addition, an order in which power of the constituent modules is turned on is not limited to a specific order.

(Third Embodiment)

An object information acquiring apparatus according to a third embodiment differs from the object information acquiring apparatus according to the first embodiment in that acoustic wave receiving elements are mounted to a hand-held probe instead of being mounted to a hemispherical supporter.

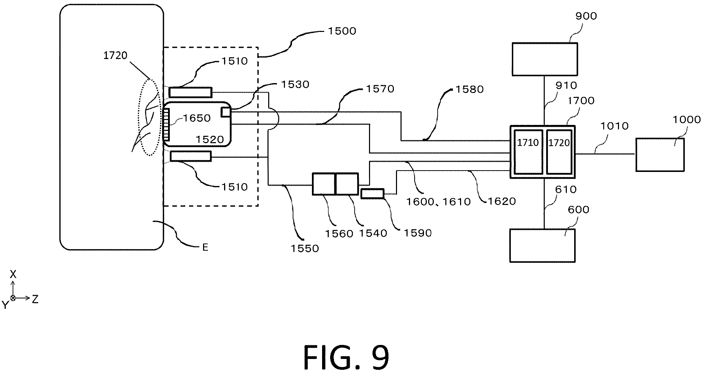

The object information acquiring apparatus according to the third embodiment will be described with reference to FIG. 9.

The object information acquiring apparatus according to the third embodiment is configured so as to include a light source 1540, an optical system 1560, a photoacoustic probe 1500, a bundle fiber 1550, and a photodetector 1590. The object information acquiring apparatus according to the third embodiment further includes the form acquiring section 600, a computer 1700, the display apparatus 900, and the input section 1000.

The photoacoustic probe 1500 is constituted by an exit end 1510, a receiving section 1520 that receives photoacoustic waves, and a scanning position sensor 1530. A plurality of acoustic wave receiving elements 1650 are arranged in the receiving section 1520.

In addition, the computer 1700 includes a calculating section 1710 and a storage section 1720.

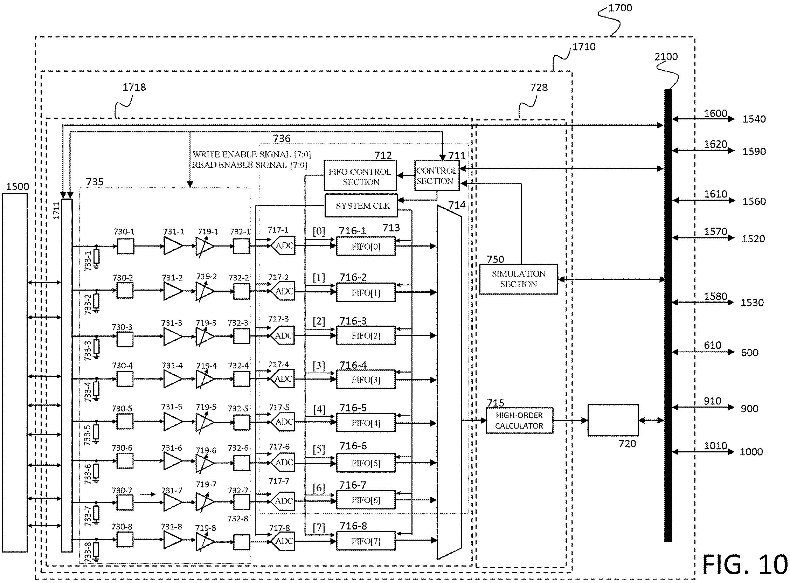

FIG. 10 is a diagram showing a configuration of the computer 1700 according to the third embodiment in greater detail. As illustrated, the calculating section 1710 is constituted by a data acquiring section 1718 and the calculation processing section 728.

The light source 1540 emits irradiation light in accordance with control by the computer 1700 and causes the irradiation light to be incident to the bundle fiber 1550 via the optical system 1560. The incident light is transferred to the photoacoustic probe 1500 and emitted from the exit end 1510.

Light irradiated to an object from the exit end 1510 disseminates inside the object, and a photoacoustic wave is emitted from a light absorber.

The plurality of acoustic wave receiving elements 1650 using piezoelectric elements, CMUTs, or the like are arranged in an array in the receiving section 1520. The photoacoustic wave emitted from the object is received by the plurality of acoustic wave receiving elements 1650, converted into an electrical signal, and sent to the computer 1700.

Moreover, while the bundle fiber 1550 is branched midway thereof and the branches are respectively connected to the two exit ends 1510 mounted so as to sandwich the receiving section 1520 in the example shown in FIG. 9, the number of branches is not limited thereto. For example, only one exit end 1510, may be provided. In addition, while the bundle fiber 1550 is used to transfer light in the present example, light may be transferred via an optical element such as a mirror or a prism.

Since the photodetector 1590 is similar to the photodetector 120 according to the first embodiment, a description thereof will be omitted.

The form acquiring section 600 according to the third embodiment adopts a circuit configuration capable of transmitting and receiving ultrasonic waves to and from the plurality of acoustic wave receiving elements 1650 arranged in the photoacoustic probe 1500, and is capable of acquiring form information of a surface (or the inside) of an object. Specifically, by transmitting an acoustic wave using a part of or all of the plurality of acoustic wave receiving elements 1650 arranged in the photoacoustic probe 1500 and analyzing a reflected wave, form information of the object is acquired.

Moreover, a process of analyzing a reflected wave may be performed by the form acquiring section 600 or by the calculation processing section 1728 included in the computer 1700.

The acquired form information is temporarily stored in a similar manner to the first embodiment.

In addition, the form acquiring section 600 has a function of transmitting and receiving acoustic waves to and from the plurality of acoustic wave receiving elements 1650 arranged in the photoacoustic probe 1500 and confirming a portion where a blood flow exists inside the object using Doppler echocardiography in a similar manner to the first embodiment. Information on the portion where a blood flow exists can be acquired and displayed two-dimensionally or three-dimensionally. Furthermore, a state of change in a blood flow can be acquired in real time in a similar manner to conventional Doppler echocardiography. Calculations pertaining to Doppler echocardiography may be performed by the form acquiring section 600 or by the computer 1700 (the calculation processing section 1728).

As described above, the photoacoustic probe 1500 according to the third embodiment is configured so as to be capable of acquiring both a photoacoustic signal and an ultrasonic echo signal (an ultrasonic signal).

A shape of the array of acoustic wave receiving elements arranged in the photoacoustic probe 1500 is not limited to a specific shape. For example, a 1D linear array, a sector array, a convex array, a 2D array, or a sparse array may be used.

In the third embodiment, both a photoacoustic wave and a reflected wave (an ultrasonic echo) are received using a plurality of acoustic wave receiving elements 1650. This can be realized by, for example, the following methods.

A first method involves using all of the plurality of acoustic wave receiving elements 1650 to receive a photoacoustic wave and an ultrasonic echo at different timings.

In addition, a second method involves assigning at least a part of the plurality of acoustic wave receiving elements 1650 to the reception of a photoacoustic wave and assigning the remaining acoustic wave receiving elements 1650 to the reception of an ultrasonic echo.

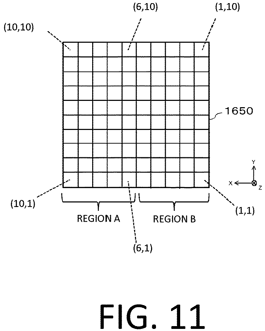

For example, FIG. 11 shows an example of a case where the plurality of acoustic wave receiving elements 1650 are arranged in a 10-element $\times$ 10-element 2D array.

For example, the reception of a photoacoustic wave and the reception of an ultrasonic echo may be respectively performed at different timings using all 100 elements. Alternatively, elements existing in a region A may be configured to receive a photoacoustic wave and elements existing in a region B may be configured to receive an ultrasonic echo.

In this manner, elements may be divided temporally or spatially. The regions can be defined in a manner different from that described above as long as the photoacoustic wave and the ultrasonic echo are received under adequate condition.

In addition, while FIG. 9 shows a mode in which a measurement is performed by bringing the photoacoustic probe 1500 into direct contact with the object, a measurement method is not limited thereto. For example, the object may be fixed by sandwiching the object with a holding member or the like and a measurement may be performed over the holding member. Alternatively, the holding member may be installed between the photoacoustic probe 1500 and the object and a measurement may be performed over the holding member. The holding member can be constituted by a resin or a gel-like member, and the holding member is desirably made of a material that favorably transmits both light and ultrasonic waves. Accordingly, the object can be measured in a stable state.

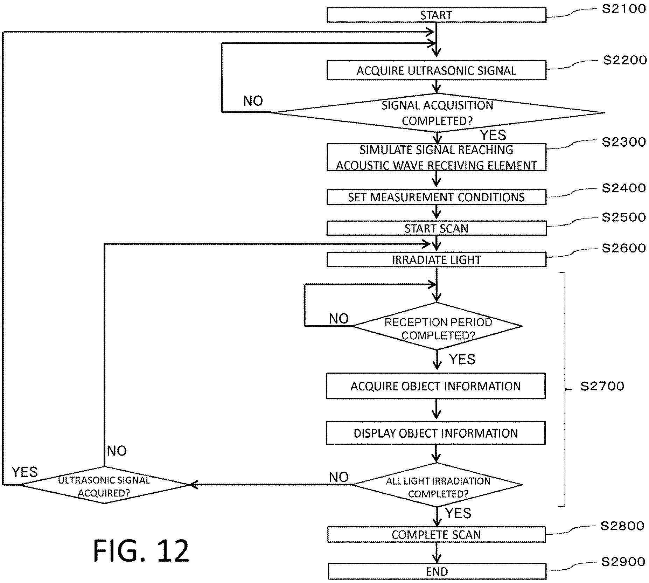

Next, operations of the object information acquiring apparatus according to the third embodiment will be described with reference to the flow chart shown in FIG. 12.

First, in step S2100, a measurement of the object is started. Moreover, in doing so, by applying a gel or the like for improving acoustic matching between the photoacoustic probe 1500 and the object, image quality of an acquirable photoacoustic image (ultrasonic echo image) can be improved.

The process of step S2200 is similar to the process of step S200 according to the first embodiment.

Moreover, a method other than Doppler imaging can be used as long as information related to a blood vessel profile inside the object can be acquired. For example, form information of the inside and outside of the object acquired in advance by another modality (X-ray CT, MRI, and the like) may be acquired.

In addition, the number of times an ultrasonic echo signal is acquired is not limited to a specific number of times. Any kind of setting is possible insofar as photoacoustic measurement is not affected.

The processes of steps S2300 and S2400 are respectively similar to the processes of steps S300 and S400 according to the first embodiment.

In the first embodiment, the processes are performed under the assumption that a photoacoustic wave originating from a surface (skin) of an object and a photoacoustic wave originating from a surface blood vessel inside the object reach an acoustic wave receiving element at the same time.

However, in the third embodiment, such a case is unlikely to occur since an acoustic wave receiving element is brought into direct contact with the surface of the object.

Nevertheless, even in the third embodiment, this does not apply when a measurement is performed by sandwiching a member having or exceeding a certain thickness between the acoustic wave receiving elements 1650 and the object. In such a case, since a situation where a photoacoustic wave originating from a surface (skin) of the object and a photoacoustic wave originating from a surface blood vessel inside the object reach the acoustic wave receiving elements 1650 at the same time may occur, setting conditions for receiving photoacoustic waves and a method of processing after reception are favorably taken into consideration.

The processes of steps S2500 and S2600 are respectively similar to the processes of steps S500 and S600 according to the first embodiment.

Although the process of step S2700 is similar to the process of step S700 according to the first embodiment, the present embodiment differs from the first embodiment in that, every time light irradiation is performed, the calculating section 1710 acquires and displays object information.

Once data acquisition under measurement conditions set in advance is completed, the scan is completed (step S2800) and the process ends (step S2900).

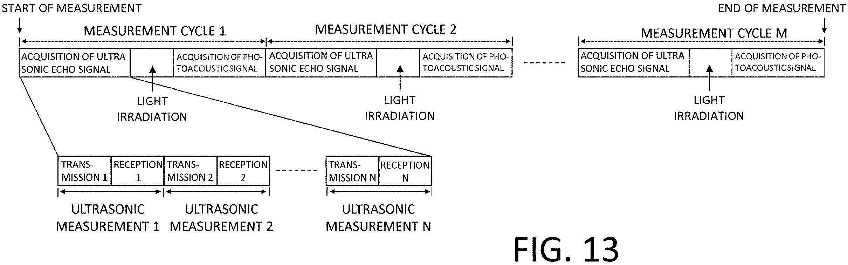

In the third embodiment, a measurement of an ultrasonic echo and photoacoustic measurement are performed in a time-shared manner. A description thereof will now be given with reference to FIG. 13. FIG. 13 is a diagram showing an order of acquisition of an ultrasonic echo signal, light irradiation, and acquisition of a photoacoustic signal.

When a measurement starts, acquisition of an ultrasonic echo signal for the purpose of acquiring form information of the inside and outside of an object is performed. Ultrasonic measurements are performed N-number (where N ≥ 0) of times, and transmission and reception of ultrasonic waves are executed in one ultrasonic measurement. An ultrasonic echo image in accordance with a width, a depth, and the number of frames set by the user is acquired from an ultrasonic echo signal obtained by the ultrasonic measurement. Photoacoustic measurement (light irradiation and acquisition of a photoacoustic signal) is performed based on a result of analyzing the ultrasonic echo image. The series of operations described above will be defined as one measurement cycle.

Moreover, while transmission and reception of ultrasonic waves are respectively performed N-number (where N ≥ 0) of times in one measurement cycle in the present example, a value of N is not limited to a specific number as long as the acquisition of a photoacoustic signal is not affected. In addition, N need not be the same among respective measurement cycles.

In the third embodiment, by repeating the measurement cycle described above, information necessary for accurately performing a photoacoustic measurement can be acquired in real time.

Moreover, in photoacoustic tomography, irradiation intervals of light must be set to a certain period of time (several 10 milliseconds) or more in order to prevent temperature of the object from rising excessively. Therefore, a frame rate of a photoacoustic image is not affected as long as an ultrasonic measurement can be completed within the irradiation intervals of light.

Moreover, while FIG. 13 shows a photoacoustic measurement being performed after performing an ultrasonic measurement, the measurements need not necessarily be performed in this order. For example, the number of ultrasonic measurements can be set to zero in each measurement cycle. For example, in a certain measurement cycle, an ultrasonic measurement may be omitted and only a photoacoustic measurement may be performed.

In addition, while an ultrasonic measurement is performed in each cycle in the present example, conditions for processing a photoacoustic signal need not be updated every time. For example, the conditions may be updated at a timing specified by the user. Therefore, for example, a button or the like may be provided in the input section 1000 or on the photoacoustic probe 1500 and conditions may be updated in accordance with input.

Moreover, while an example in which object information is acquired and displayed with every individual light irradiation has been described in the present embodiment, this example is not restrictive. For example, digital signals acquired by a plurality of light irradiations may be accumulated and object information may be collectively acquired. For example, a configuration may be adopted which allows the number of times light irradiation is to be performed before acquiring object information to be set.

In addition, an acquired photoacoustic signal may be temporarily stored and acquisition and display of object information may be performed after the scan is completed. Accordingly, an elaborate reconstructed image which requires a prolonged calculation period and which cannot be displayed in real-time can be obtained.

A configuration of the computer 1700 according to the third embodiment will now be described with reference to FIG. 10. Moreover, while eight systems of receiving circuits are provided in the example shown in FIG. 10, the number of receiving circuits is not limited thereto.

The digital circuit section 736 and the calculation processing section 728 are similar to those in the first embodiment and descriptions thereof will be omitted, and a difference between the data acquiring section 1718 according to the third embodiment and the data acquiring section 718 according to the first embodiment will now be described.

The data acquiring section 1718 differs from the data acquiring section 718 according to the first embodiment in that the data acquiring section 1718 includes a connection switch 1711. Other constituents are similar to those described in the first embodiment.

The connection switch 1711 is means for switching among connections between the photoacoustic probe 1500 and an analog circuit section 1735. Specifically, a desired acoustic wave receiving element among the plurality of acoustic wave receiving elements 1650 arranged in the photoacoustic probe 1500 can be selected and the selected acoustic wave receiving element can be connected to the analog circuit section 1735. The connection switch 1711 may be omitted depending on the design of the system.

Moreover, while FIG. 13 shows the data acquiring section 1718 being configured to acquire an ultrasonic signal and a photoacoustic signal with a same circuit, this configuration is not necessarily restrictive. The acquisition of an ultrasonic signal and the acquisition of a photoacoustic signal may be configured to be performed by different circuits depending on system specification requirements.

In the third embodiment, the form acquiring section 600 transfers a transmission waveform voltage for transmitting an ultrasonic wave to the photoacoustic probe 1500 via a wiring 610, a bus 2100, and the connection switch 1711. Accordingly, an ultrasonic wave can be transmitted by selecting a specific acoustic wave receiving element among the plurality of acoustic wave receiving elements 1650.

Moreover, while an example in which a scan is manually performed with a hand-held probe is described in the present embodiment, for example, a scan may be mechanically performed by moving a probe along a guide.

(Modifications)

It is to be understood that the descriptions of the respective embodiments merely present examples of the present invention and, as such, the present invention can be implemented by appropriately modifying or combining the embodiments without departing from the spirit and the scope of the invention.

For example, the present invention may be implemented as an object information acquiring apparatus or an information processing apparatus which includes at least a part of the processes described above. In addition, the present invention may also be implemented as an object information acquiring method or an information processing method which includes at least a part of the processes described above. The processes and units described above may be implemented in any combination thereof insofar as technical contradictions do not occur.

In addition, as described in the second embodiment, a measurement of system noise may be performed manually or may be performed automatically at a prescribed timing (for example, upon startup of the apparatus or at a set timing). Alternatively, a measurement of system noise may be performed based on an instruction from the outside. For example, a module for remote access may be connected to the bus 2000 or the bus 2100 and a measurement may be executed remotely via the module. Accordingly, maintainability is improved. Furthermore, information on the measured system noise may be accumulated and stored in the object information acquiring apparatus so as to be referable at any time.

In addition, information other than that related to system noise may be remotely transmitted. For example, information acquired by the object information acquiring apparatus (a photoacoustic signal, an ultrasonic signal, a calculated parameter value, a photoacoustic image, an ultrasonic image, an operational state of the apparatus, maintenance information, and the like) may be stored so as to be transmittable wirelessly or via a network. Furthermore, operations of the apparatus may be enabled wirelessly or via a network.

In addition, while a surface blood vessel is cited as a measurement target region (a region of interest) in the description of the embodiments, the measurement target need not necessarily be a surface blood vessel.

Furthermore, while an example of measuring oxygen saturation as functional information is cited in the description of the embodiments, functional information need not necessarily be oxygen saturation. The present invention is applicable to any substance which has a different absorption coefficient with respect to a different wavelength of an electromagnetic wave and which can be determined by a photoacoustic technique such as glycosylated protein, glucose, fat, cholesterol, and collagen.

(Other embodiments)

Embodiment(s) of the present invention can also be realized by a computer of a system or apparatus that reads out and executes computer executable instructions (e.g., one or more programs) recorded on a storage medium (which may also be referred to more fully as a 'non-transitory computer-readable storage medium') to perform the functions of one or more of the above-described embodiment(s) and/or that includes one or more circuits (e.g., application specific integrated circuit (ASIC)) for performing the functions of one or more of the above-described embodiment(s), and by a method performed by the computer of the system or apparatus by, for example, reading out and executing the computer executable instructions from the storage medium to perform the functions of one or more of the above-described embodiment(s) and/or controlling the one or more circuits to perform the functions of one or more of the above-described embodiment(s). The computer may comprise one or more processors (e.g., central processing unit (CPU), micro processing unit (MPU)) and may include a network of separate computers or separate processors to read out and execute the computer executable instructions. The computer executable instructions may be provided to the computer, for example, from a network or the storage medium. The storage medium may include, for example, one or more of a hard disk, a random-access memory (RAM), a read only memory (ROM), a storage of distributed computing systems, an optical disk (such as a compact disc (CD), digital versatile disc (DVD), or Blu-ray Disc (BD)™), a flash memory device, a memory card, and the like.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

**Claims**

1. An object information acquiring apparatus, comprising:

a signal acquiring unit configured to acquire a signal obtained by converting, with an acoustic element, an acoustic wave generated from an object to which light is irradiated;

an information acquiring unit configured to perform, on the acquired signal, a signal process including at least any of an input impedance matching process, a filtering process, an amplification process, a subtraction process, and a signal extraction process, and acquire characteristic information inside the object using the processed signal; and

a form acquiring unit configured to acquire form information of the object, wherein

the information acquiring unit determines, based on the form information of the object, contents of the signal process to be performed on a signal corresponding to an acoustic wave arriving from a target region in which the characteristic information is to be acquired.

2. The object information acquiring apparatus according to claim 1, wherein

the signal process includes a process of amplifying a signal acquired by the signal acquiring unit, and

the information acquiring unit determines, based on the form information of the object, an amplification factor of the signal within a range in which information is not lost from a signal corresponding to an acoustic wave arriving from the target region.

3. The object information acquiring apparatus according to claim 1 or 2, wherein

the target region is a region including a surface blood vessel in the object, and

the form information is information representing a position of the surface blood vessel.

4. The object information acquiring apparatus according to claim 1 or 2, wherein

the form information is information representing an external shape of the object.

5. The object information acquiring apparatus according to claim 4, wherein

the target region is a region including a surface blood vessel in the object, and

the information acquiring unit estimates a position of the surface blood vessel based on the information representing the external shape of the object.

6. The object information acquiring apparatus according to any one of claims 1 to 5, wherein

the form acquiring unit acquires the form information by transmitting an ultrasonic wave to the object and analyzing the ultrasonic wave reflected by the object.

7. The object information acquiring apparatus according to any one of claims 1 to 6, wherein

the information acquiring unit performs a simulation of an acoustic wave arriving from the target region based on the form information, and extracts a signal corresponding to an acoustic wave arriving from the target region based on a result of the simulation.

8. The object information acquiring apparatus according to any one of claims 1 to 7, wherein

the signal process is at least one of an input impedance matching process, a filtering process, an amplification process, and a subtraction process performed on an analog signal converted by the acoustic element, a filtering process, an amplification process, and a signal extraction process performed on a digital signal obtained by further converting an analog signal that has been converted by the acoustic element.

9. An object information acquiring method, comprising the steps of:

acquiring a signal obtained by converting, with an acoustic element, an acoustic wave generated from an object to which light is irradiated

performing, on the acquired signal, a signal process including at least any of an input impedance matching process, a filtering process, an amplification process, a subtraction process, and a signal extraction process, and acquiring characteristic information inside the object using the processed signal; and

acquiring form information of the object, wherein

in the step of acquiring information, contents of the signal process to be performed on a signal corresponding to an acoustic wave arriving from a target region in which the characteristic information is to be acquired is determined based on the form information of the object.

10. The object information acquiring method according to claim 9, wherein

the signal process includes a process of amplifying a signal acquired in the step of acquiring a signal, and

in the step of acquiring information, an amplification factor of the signal is determined based on the form information of the object, within a range in which information is not lost from a signal corresponding to an acoustic wave arriving from the target region.

**11.** The object information acquiring method according to claim 9 or 10, wherein
the target region is a region including a surface blood vessel in the object, and
the form information is information representing a position of the surface blood vessel.

**12.** The object information acquiring method according to claim 9 or 10, wherein
the form information is information representing an external shape of the object.

**13.** The object information acquiring method according to claim 12, wherein
the target region is a region including a surface blood vessel in the object, and
in the step of acquiring information, a position of the surface blood vessel is estimated based on the information representing the external shape of the object.

**14.** The object information acquiring method according to any one of claims 9 to 13, wherein
in the step of acquiring a form, the form information is acquired by transmitting an ultrasonic wave to the object and analyzing the ultrasonic wave reflected by the object.

**15.** A non-transitory computer readable storing medium recording a computer program for causing a computer to perform the object information acquiring method according to any one of claims 9 to 14.

FIG. 1

FIG. 2

| | |
|---|---|
| START | S100 |
| ACQUIRE PROFILE INFORMATION OF OBJECT E | S200 |
| SIMULATE SIGNAL REACHING ACOUSTIC WAVE RECEIVING ELEMENT | S300 |
| SET MEASUREMENT CONDITIONS | S400 |
| START SCAN | S500 |
| IRRADIATE LIGHT | S600 |

RECEPTION PERIOD COMPLETED? — NO / YES

ALL LIGHT IRRADIATION COMPLETED? — NO / YES

S700

| | |
|---|---|
| COMPLETE SCAN | S800 |
| ACQUIRE OBJECT INFORMATION | S900 |
| DISPLAY OBJECT INFORMATION | S1000 |
| END | S1100 |

FIG. 3

FIG. 4A

FIG. 4B

FIG. 4C

**FIG. 5**

FIG. 6

FIG. 7

FIG. 8A

COMPUTER 700
DISPLAY 900 } POWER
INPUT SECTION 1000

FORM ACQUIRING SECTION 600
POWER

SCANNER 500 POWER

LIGHT SOURCE 100 POWER

LIQUID SUPPLYING/DISCHARGING
UNIT 810   POWER

Tn0   Tn1   Tn2        Tn3        Tn4   Tn5  Tn6  Tn7

Phase A

Phase B

# FIG. 8B

FIG. 9

FIG. 10

FIG. 11

START — S2100

ACQUIRE ULTRASONIC SIGNAL — S2200

NO ← SIGNAL ACQUISITION COMPLETED?

YES

SIMULATE SIGNAL REACHING ACOUSTIC WAVE RECEIVING ELEMENT — S2300

SET MEASUREMENT CONDITIONS — S2400

START SCAN — S2500

IRRADIATE LIGHT — S2600

NO ← RECEPTION PERIOD COMPLETED?

YES

ACQUIRE OBJECT INFORMATION

DISPLAY OBJECT INFORMATION

} S2700

ULTRASONIC SIGNAL ACQUIRED?    NO ← ALL LIGHT IRRADIATION COMPLETED?

YES

YES

COMPLETE SCAN — S2800

END — S2900

FIG. 12

38

START OF MEASUREMENT

END OF MEASUREMENT

| MEASUREMENT CYCLE 1 | | MEASUREMENT CYCLE 2 | |
|---|---|---|---|

| ACQUISITION OF ULTRA SONIC ECHO SIGNAL | ACQUISITION OF PHO- TOACOUSTIC SIGNAL | ACQUISITION OF ULTRA SONIC ECHO SIGNAL | ACQUISITION OF PHO- TOACOUSTIC SIGNAL |

- - - - - - -

| MEASUREMENT CYCLE M | |
|---|---|

| ACQUISITION OF ULTRA SONIC ECHO SIGNAL | ACQUISITION OF PHO- TOACOUSTIC SIGNAL |

LIGHT
IRRADIATION

LIGHT
IRRADIATION

LIGHT
IRRADIATION

| TRANS- MISSION 1 | RECEPTION 1 | TRANS- MISSION 2 | RECEPTION 2 | | TRANS- MISSION N | RECEPTION N |
|---|---|---|---|---|---|---|

- - - - - - -

ULTRASONIC
MEASUREMENT 1

ULTRASONIC
MEASUREMENT 2

ULTRASONIC
MEASUREMENT N

FIG. 13

Europäisches
Patentamt

European
Patent Office

Office européen
des brevets

# EUROPEAN SEARCH REPORT

Application Number

EP 18 00 0013

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/058294 A1 (SUZUKI KOICHI [JP]) 3 March 2016 (2016-03-03) * abstract * * paragraphs [0033], [0057], [0120] * * the whole document * ----- | 1-15 | INV. A61B5/00 |
| A | US 2013/006088 A1 (MIYASATO TAKURO [JP]) 3 January 2013 (2013-01-03) * paragraphs [0046] - [0049] * ----- | 7 | |

TECHNICAL FIELDS
SEARCHED      (IPC)

A61B

The present search report has been drawn up for all claims

| Place of search | Date of completion of the search | Examiner |
|---|---|---|
| The Hague | 8 May 2018 | Furlan, Stéphane |

EPO FORM 1503 03.82 (P04C01)

## ANNEX TO THE EUROPEAN SEARCH REPORT
## ON EUROPEAN PATENT APPLICATION NO.

EP 18 00 0013

08-05-2018

| Patent document cited in search report | | Publication date | Patent family member(s) | | Publication date |
|---|---|---|---|---|---|
| US 2016058294 | A1 | 03-03-2016 | CN | 106175666 A | 07-12-2016 |
| | | | JP | 2016047125 A | 07-04-2016 |
| | | | US | 2016058294 A1 | 03-03-2016 |
| US 2013006088 | A1 | 03-01-2013 | BR | 112012021776 A2 | 17-05-2016 |
| | | | CN | 102843960 A | 26-12-2012 |
| | | | CN | 104644126 A | 27-05-2015 |
| | | | CN | 104644127 A | 27-05-2015 |
| | | | EP | 2552299 A1 | 06-02-2013 |
| | | | JP | 5675142 B2 | 25-02-2015 |
| | | | JP | 2011206192 A | 20-10-2011 |
| | | | KR | 20120126109 A | 20-11-2012 |
| | | | RU | 2012145885 A | 10-05-2014 |
| | | | US | 2013006088 A1 | 03-01-2013 |
| | | | WO | 2011122382 A1 | 06-10-2011 |

EPO FORM P0459

For more details about this annex : see Official Journal of the European Patent Office, No. 12/82

**EP 3 348 184 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011229815 A